# FASCICULE DE BREVET EUROPEEN

(11) **EP 1 317 943 B1**
(45) Date de publication et mention de la délivrance du brevet: **16.03.2005**
(21) Numéro de dépôt: 02293017.6
(22) Date de dépôt: 06.12.2002
(51) Int. Cl.: A61N 1/365, A61N 1/36

(54) **Dispositif médical actif comprenant des moyens perfectionnés de discrimination des phases d'éveil et de sommeil**
Aktive implantierbare medizinische Vorrichtung mit verbesserten Mitteln zum Unterscheiden zwischen den Phasen von Wachzustand und Schlafzustand
Active implantable medical device comprising improved means for discriminating between the phases of arousal and sleep

(30) Priorité: 07.12.2001 FR 0115867
(43) Date de publication de la demande: 11.06.2003
(73) Titulaire: ELA MEDICAL, F-92541 Montrouge (FR)
(72) Inventeur: Poezevera, Yann, 91080 Courcouronnes (FR); Limousin, Marcel, 75014 Paris (FR)
(74) Mandataire: Dupuis-Latour, Dominique

(56) Documents cités:
- EP-A- 0 719 568
- EP-A- 0 940 155
- US-A- 5 476 483
- US-B1- 6 188 927

## Description

L'invention concerne les "dispositifs médicaux actifs" tels que définis par la directive 90/385/CEE du 20 juin 1990 du Conseil des communautés européennes.

Elle sera plus particulièrement décrite dans le cas de dispositifs implantables tels que des stimulateurs cardiaques, défibrillateurs et/ou cardioverteurs permettant de délivrer au coeur des impulsions électriques de faible énergie pour le traitement des troubles du rythme cardiaque. On soulignera cependant que ce type de dispositif n'est en aucune façon limitatif de l'invention et que les enseignements de cette dernière sont directement applicables à de nombreux types de dispositifs médicaux actifs, de diagnostic et/ou de thérapie.

L'invention concerne plus particulièrement le diagnostic des troubles survenant pendant le sommeil, qu'il s'agisse de troubles d'ordre cardiaque ou de troubles respiratoires tels que les apnées ou hypopnées révélatrices en particulier d'une pathologie connue sous le nom de "syndrome d'apnée du sommeil (SAS)". Une apnée est définie comme étant une pause respiratoire de durée supérieure à 10 secondes et survenant pendant une phase de sommeil du patient (car une apnée en état d'éveil ne peut en aucun cas être à l'origine d'un SAS).

Le diagnostic de ces troubles implique que le dispositif puisse discriminer le mieux possible entre phases d'éveil et phases de sommeil, l'analyse du rythme respiratoire et/ou cardiaque ne devant bien entendu être effectuée en vue de ce diagnostic que pendant les phases de sommeil.

L'importance de cette discrimination exacte entre sommeil et éveil est rendue encore plus nécessaire lorsque le dispositif non seulement opère un diagnostic mais applique une thérapie : cette thérapie ne doit être appliquée que pendant les phases de sommeil, et tout traitement doit être inhibé si l'apnée survient pendant une phase d'éveil, car dans ce cas elle n'est normalement pas pathologique.

D'après le EP-A-0 719 568 (Ela Médical), il est connu d'opérer la discrimination entre éveil et sommeil par analyse d'un signal physiologique de ventilation-minute (MV) représentatif de la périodicité et de l'amplitude des cycles respiratoires successifs du patient, la ventilation-minute étant définie comme le produit de l'amplitude par la fréquence respiratoires.

Plus précisément, le EP-A-0 719 568 propose d'acquérir une série d'échantillons successifs du signal MV et d'en calculer une moyenne sur un nombre donné de cycles respiratoires, par exemple les 128 derniers cycles, et de comparer cette valeur moyenne avec une valeur de référence, par exemple la moyenne du signal MV sur les dernières 24 heures.

En effet, la variation circadienne de la fréquence et de l'amplitude des cycles respiratoires est bien reproduite par le signal MV. Le calcul de la ventilation moyenne sur 24 heures permet donc d'opérer une discrimination satisfaisante entre une ventilation d'éveil et une ventilation de sommeil.

Toutefois, le signal MV présente une variabilité naturelle en raison de la nature à la fois végétative et contrôlée du système respiratoire. Ainsi, les soupirs, les apnées volontaires au cours de la parole ou encore les apnées du sommeil font qu'une mesure instantanée n'est pas représentative du niveau courant effectif de la ventilation-minute, c'est-à-dire du niveau permettant d'analyser l'état d'activité du patient.

C'est pour cette raison que le diagnostic d'éveil ou de sommeil ne peut être opéré de façon fiable que si l'on détermine la ventilation-minute courante par moyennage d'un nombre relativement élevé de cycle respiratoire, typiquement les 128 cycles derniers cycles respiratoires, ceci pour éliminer la variation naturelle mais également les artefacts liés à la chaîne de mesure.

La conséquence de cette manière de procéder est que, lorsque l'activité respiratoire passe d'un état de sommeil à un état d'éveil, le système de détection présente un certain retard au diagnostic d'éveil, puisqu'il faut attendre que la valeur moyenne du signal MV devienne supérieure au seuil permettant de discriminer entre éveil et sommeil ; le problème se pose de la même façon lors du passage de l'état d'éveil à l'état de sommeil.

Dans certains cas, ce retard n'est pas gênant. Par exemple, la diminution progressive de la fréquence de stimulation cardiaque au cours du sommeil, destinée à respecter la physiologie naturelle du patient, ne requiert pas une réactivité importante.

En revanche, la surveillance d'un événement ou le suivi d'un paramètre survenant exclusivement au cours du sommeil (trouble respiratoire tel qu'apnée du sommeil, événement cardiaque particulier) peut nécessiter une grande réactivité afin de ne pas manquer cet événement. Ceci est particulièrement important lorsque le patient a un sommeil déstructuré et qu'il présente de nombreux réveils au cours de la nuit, surtout si ces réveils sont, précisément, causés par les événements que l'on cherche à détecter (un exemple typique en étant les apnées du sommeil).

De plus, si l'incidence du paramètre analysé est calculée à partir du temps de sommeil total (comme dans le cas du calcul de l'indice d'apnée du sommeil, qui est défini comme étant le nombre d'apnées par heure de sommeil), il est d'autant plus nécessaire d'identifier rapidement les transitions entre phases d'éveil et de sommeil pour évaluer correctement le temps de sommeil total et opérer un diagnostic correct. Tel est par exemple le cas lorsque l'on définit qu'il y a SAS lorsque l'indice d'apnée dépasse un seuil prédéterminé, par exemple plus de dix apnées par heure de sommeil.

L'invention a pour but de pallier ces divers inconvénients dus au retard du diagnostic d'éveil ou de sommeil lors des changements de phases, retard résultant de la nécessité de moyenner le signal MV sur un nombre relativement élevé de cycles respiratoires.

On soulignera que l'invention n'est pas limitée aux dispositifs procédant par analyse d'un signal physiologique de ventilation-minute, ni même d'un signal représentatif de l'activité respiratoire d'un patient. Il s'agit là de la configuration la plus courante, et la présente description sera faite dans le cadre de cet exemple. Toutefois, l'invention s'applique aussi bien à des dispositifs mettant en oeuvre d'autres types de capteurs physiologiques à évolution lente tels que capteurs de température ou de pH, capteurs de mesure de la saturation en oxygène du sang, etc.

De façon générale, l'invention a pour but d'améliorer la réactivité du dispositif aux changements de phases éveil/sommeil à des fins d'amélioration du diagnostic, pour éviter tant les faux positifs (détection d'une apnée ou d'un artefact au début d'une phase d'éveil) que les faux négatifs (non-détection d'une apnée en début de phase de sommeil).

Elle a également pour but d'améliorer les conditions de déclenchement d'une thérapie, pour éviter d'appliquer à tort pendant des phases d'éveil des traitements qui ne doivent l'être exclusivement que pendant des phases de sommeil.

L'idée de base de l'invention repose sur l'utilisation d'un capteur auxiliaire à réponse rapide, typiquement un capteur d'activité ou d'accélération ("capteur G"), dont le signal permet de détecter des mouvements du patient. Contrairement au capteur MV, l'information de ce type de capteur n'est pas très spécifique des phases d'éveil ou de sommeil ; en revanche sa réponse est rapide, de sorte que l'on peut utiliser le signal délivré par ce capteur auxiliaire pour améliorer la dynamique du capteur MV lors des changements de phase, même si le diagnostic des phases d'éveil ou de sommeil reste uniquement fondé sur le signal délivré par le capteur MV.

Il est certes connu d'utiliser conjointement les signaux délivrés par deux capteurs, à savoir un capteur d'effort à réponse lente mesurant un paramètre à prépondérance physiologique (typiquement un capteur MV), et un capteur d'activité à faible temps de réponse mesurant un paramètre à prépondérance physique (typiquement un capteur G).

Les EP-A-0 750 920 (Ela Médical) et EP-A-0 770 407 (Ela Médical) décrivent des dispositifs mettant en oeuvre une telle combinaison de capteurs, mais dans des buts différents, par exemple pour adapter l'asservissement d'une stimulation cardiaque à la situation physiologique réelle du patient, (EP-A-0 750 920) ou encore pour mettre hors service dans certaines situations une chaîne de mesure afin de réduire la consommation énergique du dispositif (EP-A-0 770 407).

Dans ces documents, la combinaison des capteurs n'a pas pour objet d'opérer une discrimination entre des phases d'éveil et de sommeil, encore moins de modifier, dans ce contexte, l'analyse d'un signal MV en fonction de l'information délivrée par un capteur d'activité.

Plus précisément, le dispositif médical de l'invention est du type général décrit dans le EP-A-0 719 568 précité, c'est-à-dire comprenant : des moyens de mesure d'un paramètre corporel d'un patient, délivrant un signal physiologique, ainsi que des moyens de détection des phases d'éveil et de sommeil du patient, comprenant des moyens pour établir une moyenne des valeurs successives du signal physiologique, calculée sur une durée antérieure prédéterminée, des premiers moyens comparateurs, pour comparer ladite moyenne à un seuil physiologique prédéterminé, et des moyens pour indiquer un premier état d'éveil lorsque ladite moyenne est supérieure audit seuil physiologique, et pour indiquer un premier état de sommeil dans le cas contraire.

Selon l'invention, ce dispositif comprend également : des moyens de mesure d'activité, aptes à délivrer un signal physique d'activité du patient à variation plus rapide que le signal physiologique ; des seconds moyens comparateurs, pour comparer le signal d'activité à un seuil d'activité prédéterminé ; et des moyens pour indiquer un second état d'éveil lorsque ledit signal d'activité est supérieur audit seuil d'activité, et pour indiquer un second état de sommeil dans le cas contraire. Par ailleurs, lesdits moyens de détection des phases d'éveil et de sommeil du patient comprennent également : des moyens pour comparer ledit premier état d'éveil ou de sommeil avec ledit second état d'éveil ou de sommeil ; et des moyens anticipateurs, pour modifier sélectivement ladite durée antérieure prédéterminée en cas de discordance entre lesdits premier et second états d'éveil ou de sommeil.

Le signal physiologique peut en particulier être un signal de ventilation-minute, ladite durée antérieure prédéterminée étant une succession de cycles respiratoires antérieurs.

Très avantageusement, les moyens anticipateurs sont des moyens aptes à modifier, notamment à réduire, en particulier à réduire d'au moins 50 %, ladite durée antérieure prédéterminée sur laquelle est calculée ladite moyenne des valeurs successives du signal physiologique.

Les moyens anticipateurs peuvent, plus précisément, être des moyens aptes à sélectivement réduire ladite durée antérieure prédéterminée lorsque ledit premier état est un état d'éveil, ledit second état est un état de sommeil, et la valeur de ladite moyenne évolue dans un sens décroissant, ou bien lorsque ledit premier état est un état de sommeil, ledit second état est un état d'éveil, et la valeur de ladite moyenne évolue dans un sens stable ou croissant.

Le signal physique d'activité du patient est avantageusement un signal délivré par un capteur d'accélération.

L'invention s'applique très préférentiellement au cas où le dispositif comprend des moyens de détection de troubles respiratoires du sommeil, notamment de détection d'apnées et de calcul d'un indice d'apnée, ces moyens n'étant alors activés que lorsque ledit premier état est un état de sommeil.

L'invention peut en particulier être mise en oeuvre dans un dispositif implantable actif du type stimulateur cardiaque, défibrillateur, cardioverteur et/ou dispositif multisite, comprenant des moyens de stimulation de l'activité cardiaque, ces moyens opérant en réponse au signal physiologique mesuré et à la détection des phases d'éveil et de sommeil du patient.

On va maintenant décrire un exemple de mise en oeuvre de l'invention, en référence à la figure unique annexée.

Cette figure montre une série de chronogrammes expliquant la manière dont la discrimination entre éveil et sommeil est opérée conformément aux enseignements de la présente invention.

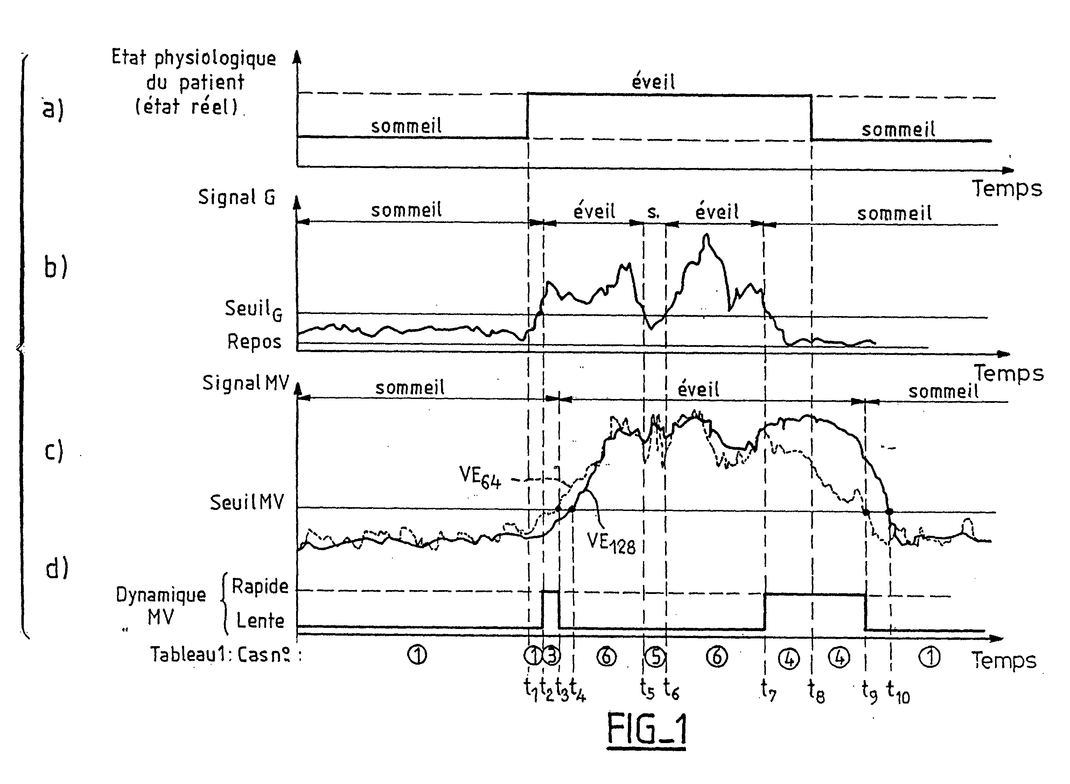

Sur la figure 1, on a représenté sur le chronogramme de la ligne (a) l'état physiologique, réel, du patient, qui est initialement un état de sommeil. À l'instant t₁ le patient se réveille, et cette période d'éveil s'achève à l'instant t₈, où le patient entre dans une nouvelle phase de sommeil.

Sur le chronogramme de la ligne (c), on a représenté en trait plein le signal MV délivré par le capteur de ventilation-minute, après que ce signal ait été échantillonné et moyenné sur les 128 cycles respiratoires précédents. Cette valeur moyenne est désignée VE₁₂₈.

Le signal MV est un paramètre à prépondérance physiologique obtenu par une mesure intrathoracique d'impédance. Cette mesure est opérée entre deux électrodes disposées dans la cage thoracique, ou entre une électrode (par exemple une électrode de stimulation, si le dispositif implanté est un stimulateur cardiaque) et un boîtier du dispositif. L'impédance est mesurée par injection d'un courant constant de quelques centaines de milliampères, à une fréquence de quelques Hertz, typiquement 8 Hz. Cette technique est par exemple décrite par Bonnet JL et coll., Measurement of Minute-Ventilation with Different DDDR Pacemaker Electrode Configurations, *PACE,* Vol. 21, 98, Part 1, et elle est mise en oeuvre dans les appareils *Chorus RM 7034* d'ELA Médical.

La période de sommeil est diagnostiquée bien entendu de façon automatique, typiquement à partir du signal délivré par le capteur de suivi du rythme respiratoire du patient. Toutefois, bien que le signal de ventilation-minute soit généralement le plus aisé à utiliser pour le suivi du rythme respiratoire du patient, d'autres signaux provenant d'autres types de capteurs peuvent être utilisés en variante ou en complément du capteur MV, par exemple un capteur de mesure de la saturation en oxygène dans le sang.

Jusqu'à présent, la transition entre états d'éveil et de sommeil était détectée en comparant la valeur moyenne VE₁₂₈ à un seuil, désigné "Seuil MV", déterminé à partir de la valeur moyenne sur 24 heures du signal MV.

Ainsi, dans l'exemple illustré, le réveil du patient était détecté à l'instant t₄ (donc avec un retard t₄-t₁ par rapport à l'instant réel du réveil) et son endormissement à l'instant t₁₀ (donc avec un retard t₁₀-t₈ par rapport à l'instant réel d'endormissement).

Pour réduire ces retards à la détection des changements de phases, l'invention propose d'utiliser un capteur auxiliaire, typiquement un capteur d'accélération ("capteur G").

Le signal délivré directement par le capteur est moyenné sur une durée relativement courte (par exemple 64 cycles cardiaques) afin d'éliminer les artefacts et les brèves variations non significatives. Ce signal moyenné, désigné "signal G" a été représenté par la courbe du chronogramme de la ligne (b) de la figure 1.

Ce signal G est comparé à un seuil, désigné "Seuil G", qui est par exemple fixé à 10 % au-dessus de la valeur du seuil de base. Si Signal G dépasse Seuil G, on définira un état d'éveil selon le capteur G ; dans le cas contraire, on définira un état de sommeil selon le capteur G.

Le dispositif dispose donc de deux indicateurs d'éveil/sommeil, définis à partir des deux signaux Signal MV et Signal G.

Ces deux états peuvent être concordants ou non.

L'état d'éveil ou de sommeil du patient continue à être diagnostiqué sur la base du signal MV mais, selon le cas, la valeur devant être comparée au seuil MV sera soit la moyenne VE₁₂₈ (on parlera alors de "dynamique lente") soit la moyenne VE₆₄, calculée sur une période plus courte, typiquement sur les 64 échantillons précédents (on parlera alors de "dynamique rapide").

L'évolution de la moyenne VE₆₄ est illustrée en trait interrompu sur le chronogramme de la ligne (c) de la figure 1, où l'on peut voir que la caractéristique présente une forme plus accidentée que celle de la moyenne VE₁₂₈, en raison du moyennage sur une période plus courte entraînant une variabilité plus grande.

Si les états (éveil/sommeil) donnés par les deux signaux Signal G et Signal MV concordent, alors le fonctionnement du dispositif n'est pas modifié, c'est-à-dire que l'état d'éveil ou de sommeil est déterminé à partir du signal MV en comparant VE₁₂₈ à Seuil MV (dynamique lente).

En revanche, en cas de discordance entre les deux signaux, un critère supplémentaire est introduit, qui est la tendance du signal MV : décroissante, stable ou croissante. Cette tendance est déterminée par comparaison de la valeur actuelle VE₁₂₈ avec la valeur VE₁₂₈ précédemment calculée ; la tendance est dite stable si l'écart est inférieur à 10 %, sinon elle est dite croissante ou décroissante, selon le signe de l'écart.

Lorsqu'un changement d'état du capteur G survient, et que le signal MV indique une tendance appropriée, le fonctionnement du dispositif est modifié de manière à déterminer l'état d'éveil ou de sommeil non plus à partir de VE₁₂₈ (dynamique lente), mais à partir de VE₆₄ (dynamique rapide) de manière à procurer une plus grande réactivité.

Les cas de figures où la dynamique est rendue rapide sont résumés par la table de vérité ci-dessous.

**Tableau 1**

| État capteur MV | État capteur G | Tendance Signal MV | Dynamique | Cas N° |
|---|---|---|---|---|
| sommeil | sommeil | - | lente | ① |
| sommeil | éveil | décr./stable | lente | ② |
| | | croissante | rapide | ③ |
| éveil | sommeil | décroissante | rapide | ④ |
| | | croiss./stable | lente | ⑤ |
| éveil | éveil | - | lente | ⑥ |

Si l'on se reporte à l'exemple illustré sur la figure 1, initialement le capteur MV indique un état de sommeil (VE₁₂₈ < Seuil MV) ; tant que le capteur G confirme cet état, la dynamique reste lente.

A l'instant t₁, le patient se réveille, mais aucun des deux capteurs n'a encore franchi le seuil définissant le changement d'état.

A l'instant t₂, cet éveil est diagnostiqué par le capteur G, et comme la tendance du signal MV est croissante, l'analyse du signal MV passe à une dynamique rapide : c'est le signal VE₆₄ (et non plus VE₁₂₈) qui est alors comparé au Seuil MV.

Lorsque, à l'instant t₃, VE₆₄ atteint Seuil MV, les deux capteurs indiquent un état d'éveil, qui est donc confirmé comme tel au dispositif, et la dynamique redevient lente.

Lors de l'épisode entre les instants t₅ et t₆, qui peut par exemple correspondre à une période de bref repos, le capteur G indique un état de sommeil (Signal G repasse au-dessous de Seuil G) mais VE₁₂₈ reste supérieur à Seuil MV - donc le dispositif continue à considérer qu'il y a état d'éveil et, la tendance MV n'étant pas décroissante, la dynamique reste inchangée (elle reste lente).

La fin de la phase d'éveil est caractérisée par un repos progressif du patient, qui conduira au passage à la phase de sommeil à l'instant t₈. Pendant cette période de repos progressif, l'endormissement est détecté à l'instant t₇, par le capteur G. La tendance du signal MV étant décroissante, la dynamique devient rapide, afin de pouvoir détecter les apnées qui surviendront en début de sommeil, et être précis sur le nombre d'épisodes. Cette dynamique rapide est maintenue jusqu'à confirmation du sommeil, à l'instant t₉, par le capteur MV, correspondant au franchissement du Seuil MV par le signal VE₆₄.

En définitive, la détection des phases d'éveil ou de sommeil selon l'invention permet d'avancer l'instant de détection de la phase d'éveil de t₄ (avec la méthode antérieure) à t₃ (avec l'invention), et la détection de la phase de sommeil de t₁₀ (avec la méthode antérieure) à t₉ (avec l'invention).

L'invention permet ainsi d'améliorer de façon substantielle le diagnostic des troubles survenant pendant le sommeil (troubles respiratoires et/ou cardiaques), le comptage des événements correspondant et le calcul des paramètres tel que l'indice d'apnée, tout en conservant les avantages du calcul à partir d'une valeur du signal MV suffisamment moyennée pour éliminer les variations naturelles et les artefacts liés à la chaîne de mesure.

On notera que l'utilisation des signaux VE₁₂₈ et VE₆₄ n'est pas limitative, et qu'il est possible d'utiliser également aux mêmes fins les signaux VE64 et

VE₃₂, ou VE₁₆, etc. définis de manière comparable.

Par ailleurs, il peut être avantageux de prévoir après chaque changement de dynamique une phase consécutive de temporisation (par exemple d'une durée de x cycles respiratoires) ou incluant un cycle d'hystérésis, durant laquelle la dynamique n'est plus modifiée, ceci pour éviter les phénomènes d'oscillations indésirables lors des changements de dynamique.

## Revendications

1. Un dispositif médical actif, comprenant :
- des moyens de mesure d'un paramètre corporel d'un patient, délivrant un signal physiologique, ainsi que
- des moyens de détection des phases d'éveil et de sommeil du patient, comprenant :
. des moyens pour établir une moyenne des valeurs successives du signal physiologique, calculée sur une durée antérieure prédéterminée,
. des premiers moyens comparateurs, pour comparer ladite moyenne à un seuil physiologique prédéterminé et
. des moyens pour indiquer un premier état d'éveil lorsque ladite moyenne est supérieure audit seuil physiologique, et pour indiquer un premier état de sommeil dans le cas contraire,
dispositif **caractérisé en ce qu'**il comprend également :
- des moyens de mesure d'activité, aptes à délivrer un signal physique d'activité du patient à variation plus rapide que le signal physiologique,
- des seconds moyens comparateurs, pour comparer le signal d'activité à un seuil d'activité prédéterminé, et
- des moyens pour indiquer un second état d'éveil lorsque ledit signal d'activité est supérieur audit seuil d'activité, et pour indiquer un second état de sommeil dans le cas contraire, et
et **en ce que** lesdits moyens de détection des phases d'éveil et de sommeil du patient comprennent également :
. des moyens pour comparer ledit premier état d'éveil ou de sommeil avec ledit second état d'éveil ou de sommeil, et
. des moyens anticipateurs, pour modifier sélectivement ladite durée antérieure prédéterminée en cas de discordance entre lesdits premier et second états d'éveil ou de sommeil.

2. Le dispositif de la revendication 1, dans lequel ledit signal physiologique est un signal de ventilation-minute et ladite durée antérieure prédéterminée est une succession de cycles respiratoires antérieurs.

3. Le dispositif de la revendication 1, dans lequel lesdits moyens anticipateurs sont des moyens aptes à modifier ladite durée antérieure prédéterminée sur laquelle est calculée ladite moyenne des valeurs successives du signal physiologique.

4. Le dispositif de la revendication 3, dans lequel lesdits moyens anticipateurs sont des moyens aptes à réduire ladite durée antérieure prédéterminée.

5. Le dispositif de la revendication 4, dans lequel lesdits moyens anticipateurs sont des moyens aptes à réduire d'au moins 50 % ladite durée antérieure prédéterminée.

6. Le dispositif de la revendication 4, dans lequel lesdits moyens anticipateurs sont des moyens aptes à sélectivement réduire ladite durée antérieure prédéterminée :
- lorsque ledit premier état est un état d'éveil, ledit second état est un état de sommeil, et la valeur de ladite moyenne évolue dans un sens décroissant, ou bien
- lorsque ledit premier état est un état de sommeil, ledit second état est un état d'éveil, et la valeur de ladite moyenne évolue dans un sens stable ou croissant.

7. Le dispositif de la revendication 1, dans lequel ledit signal physique d'activité du patient est un signal délivré par un capteur d'accélération.

8. Le dispositif de la revendication 1, comprenant en outre :
- des moyens de détection de troubles respiratoires du sommeil, ces moyens n'étant activés que lorsque ledit premier état est un état de sommeil.

9. Le dispositif de la revendication 8, dans lequel les moyens de détection de troubles respiratoires du sommeil sont des moyens de détection d'apnées et de calcul d'un indice d'apnée.

10. Le dispositif de la revendication 1, dans lequel ledit dispositif est un dispositif implantable actif du type stimulateur cardiaque, défibrillateur, cardioverteur et/ou dispositif multisite, comprenant des moyens de stimulation de l'activité cardiaque, ces moyens opérant en réponse au signal physiologique mesuré et à la détection des phases d'éveil et de sommeil du patient.

## Patentansprüche

1. Aktive medizinische Vorrichtung, umfassend:
- Mittel zur Messung eines Körperparameters eines Patienten, die ein physiologisches Signal liefern, sowie
- Mittel zur Entdeckung von Wach- und Schlafphasen des Patienten, umfassend:
- Mittel zur Ermittlung eines Durchschnitts von aufeinanderfolgenden Werten des physiologischen Signals, berechnet über einen vorhergehenden vorbestimmten Zeitraum,
- erste Vergleichsmittel, um den Durchschnitt mit einer vorbestimmten physiologischen Schwelle zu vergleichen, und
- Mittel, um einen ersten Wachzustand anzuzeigen, wenn der Durchschnitt größer ist als die physiologische Schwelle, und um einen ersten Schlafzustand im gegenteiligen Fall anzuzeigen,
wobei die Vorrichtung **dadurch gekennzeichnet ist, dass** sie ebenfalls umfasst:
- Mittel zu Messung der Aktivität, dazu geeignet, ein physisches Signal der Aktivität des Patienten mit schnellerem Wechsel als das physiologische Signal zu liefern,
- zweite Vergleichsmittel, um das Signal der Aktivität mit einer vorbestimmten Schwelle der Aktivität zu vergleichen, und
- Mittel, um einen zweiten Wachzustand anzuzeigen, wenn das Signal der Aktivität größer ist als die Schwelle der Aktivität, und einen zweiten Schlafzustand im gegenteiligen Fall anzuzeigen, und
dadurch, dass die Mittel zur Entdeckung der Wach- und Schlafphasen ebenfalls umfassen:
- Mittel zum Vergleich des ersten Wach- oder Schlafzustands mit dem zweiten Wach- oder Schlafzustand, und
- vorwegnehmende Mittel zur selektiven Modifizierung des vorbestimmten vorhergehenden Zeitraums im Falle der Nicht-Übereinstimmung des ersten und des zweiten Wachoder Schlafzustands.

2. Vorrichtung gemäß Anspruch 1, in der das physiologische Signal ein Signal des Atemminutenvolumens ist und der vorbestimmte vorhergehende Zeitraum eine Folge von vorhergehenden Atemzyklen ist.

3. Vorrichtung gemäß Anspruch 1, in der die vorwegnehmenden Mittel Mittel sind, die geeignet sind, den vorbestimmten vorhergehenden Zeitraum zu verändern, über den der Durchschnitt der aufeinanderfolgenden Werte des physiologischen Signals berechnet wird.

4. Vorrichtung gemäß Anspruch 3, in der die vorwegnehmenden Mittel Mittel sind, die geeignet sind, den vorbestimmten vorhergehenden Zeitraum zu verringern.

5. Vorrichtung gemäß Anspruch 4, in der die vorwegnehmenden Mittel Mittel sind, die geeignet sind, den vorbestimmten vorhergehenden Zeitraum um mindestens 50% zu verringern.

6. Vorrichtung gemäß Anspruch 4, in der die vorwegnehmenden Mittel Mittel sind, die geeignet sind, den vorbestimmten vorhergehenden Zeitraum selektiv zu verringern:
- wenn der erste Zustand ein Wachzustand ist, der zweite Zustand ein Schlafzustand ist und der Wert des Durchschnitts sich in abnehmende Richtung entwickelt, oder
- wenn der erste Zustand ein Schlafzustand ist, der zweite Zustand ein Wachzustand ist, und der Wert des Durchschnitts sich in stabiler oder zunehmender Richtung entwickelt.

7. Vorrichtung gemäß Anspruch 1, in der das physische Signal der Aktivität des Patienten ein Signal ist, das durch einen Beschleunigungssensor geliefert wird.

8. Vorrichtung gemäß Anspruch 1, die darüber hinaus umfasst:
- Mittel zur Entdeckung von Schlaf-Atemstörungen, wobei diese Mittel nur aktiviert werden, wenn der erste Zustand ein Schlafzustand ist.

9. Vorrichtung gemäß Anspruch 8, in der die Mittel zur Entdeckung von Schlaf-Atemstörungen Mittel zur Entdeckung von Apnoe und zur Berechnung eines Apnoe-Indexes sind.

10. Vorrichtung gemäß Anspruch 1, in der die Vorrichtung eine aktive implantierbare Vorrichtung vom Typ Herzschrittmacher, Defibrillator, Kardioverter und/oder eine multizentrische Vorrichtung ist, die Mittel zur Stimulation der kardialen Aktivität umfasst, wobei die Mittel in Abhängigkeit des gemessenen physiologischen Signals und der Entdeckung der Wach- und Schlafphasen des Patienten handeln.

## Claims

1. An active medical device, comprising:
- means for measuring a physiological parameter of a patient, issuing a physiological signal; as well as
- means for detecting awakening and sleep phases of the patient, including:
· means for computing an average of the successive values of the physiological signal, computed over a prior predetermined period,
· first comparator means, for comparing said average with a predetermined physiological threshold; and
· means for indicating a first awakening state when said average is greater than said physiological threshold, and for indicating a first sleep state otherwise;
said device being **characterised by** further comprising:
- activity measurement means, adapted to issue a patient activity physical signal which varies faster than the physiological signal;
- second comparator means, for comparing the activity signal with a predetermined activity threshold; and
- means for indicating a second awakening state when said activity signal is greater than said activity threshold, and for indicating a second sleep state otherwise;
- means for comparing the first state of the patient with the second state of the patient;
and in that said means for detecting awakening and sleep phases of the patient further comprises:
- means for comparing said first awakening or sleep state to said second awakening or sleep state, and
- anticipating means, for selectively modifying said prior predetermined period in case of a discordance between said first and second awakening or sleep states.

2. The device of claim 1, wherein said physiological signal is a minute-ventilation signal and said prior predetermined period is a succession of prior respiratory cycles.

3. The device of claim 1, wherein said anticipating means is a means adapted to modify said prior predetermined period over which said average of successive values of the physiological signal is computed.

4. The device of claim 3, wherein said anticipating means is a means adapted to reduce said prior predetermined period.

5. The device of claim 4, wherein said anticipating means is a means adapted to reduce by at least 50% said prior predetermined period.

6. The device of claim 4, wherein said anticipating means is a means adapted to selectively reduce said prior predetermined period:
- either when said first state is an awakening state, said second state is a sleep state, and the value of said average changes at a decreasing trend,
- or when said first state is a sleep state, said second state is an awakening state, and the value of said average changes at a stable increasing trend.

7. The device of claim 1, wherein said patient activity physical signal is a signal issued by an acceleration sensor.

8. The device of claim 1, further comprising:
- means for detecting respiratory sleep disorders, said means being activated only when said first state is a sleep state.

9. The device of claim 8, wherein the means for detecting respiratory sleep disorders is a means for detecting apnea and for calculating an apnea index.

10. The device of claim 1, wherein said device is an active implantable device of the cardiac pacemaker, defibrillator, cardioverter, and/or multisite device type, including means operable in response to the measured physiological signal and to the detection of awakening and sleep phases of the patient.
